# DEMANDE DE BREVET EUROPEEN

(11) **EP 3 045 132 A1**
(43) Date de publication de la demande: **20.07.2016**
(21) Numéro de dépôt: 15305022.4
(22) Date de dépôt: 13.01.2015
(51) Int. Cl.: A61B 18/02, A61B 17/00

(54) **Traitement cosmétique des taches brunes cutanées**

(71) Demandeur: Starpharm, 78620 L'Etang La Ville (FR)
(72) Inventeur: Marin, Denis, 78620 L Etang La Ville (FR)
(74) Mandataire: Bugnion Genève

(57) **Abrégé**

Procédé et dispositif de traitement cosmétique de tâches cutanées comprenant une étape d'application d'un jet de fluide cryogénique, en particulier de difluoroéthane, sur la ladite zone pendant deux à quatre secondes, amenant ladite zone à une température comprise entre -4°C et - 10°C pour obtenir une action cyto-sélective.

## Description

### DOMAINE TECHNIQUE DE L'INVENTION

La présente invention concerne un procédé de traitement cosmétique de taches brunes cutanées, destiné à faire disparaître lesdites tâches brunes situées au niveau des mains, du visage, des membres et du décolleté de sujets atteints de telles hyperpigmentations cutanées.

La mélanogenèse par l'intermédiaire de cellules spécialisées appelées mélanocytes, à l'origine de la coloration de la peau, est influencée par des facteurs extérieurs qui entraînent une augmentation de la production de mélanine et, par voie de conséquence, une coloration localisée plus importante. Il y a alors formation de taches pigmentaires.

Les taches brunes (lentigos, dermatites actiniques) peuvent apparaître à partir de 30 ans, parfois dès l'adolescence, et sont localisées principalement sur les mains, le visage et le décolleté. Elles sont la conséquence de l'exposition trop importante au soleil. L'héliodermie désigne l'ensemble des conséquences du soleil sur la peau, tel que les taches, le relâchement de la peau, l'apparition de rides, avec un aspect fripé, de peau sèche. Elle peut se manifester au niveau des taches par:
- éphélides ou « taches de rousseur » dont le nombre et l'intensité augmentent sous l'effet de l'exposition aux ultraviolets ;
- lentigos solaires des zones surexposées au soleil ;
- hyperchromie (pigmentation anormale) de type mélasma dont l'intensité est directement liée à l'exposition aux ultraviolets A et B ;
- « dermite en breloque » qui est une séquelle d'une photosensibilisation induite par un parfum.

Le vieillissement cutané intrinsèque entraine une atrophie qui s'accompagne de phénomènes de relâchement cutané, de dessèchement et de troubles pigmentaires. Le principal trouble pigmentaire est le lentigo sénile, qui apparaît vers la cinquantaine sur les parties découvertes de la peau, et plus particulièrement sur le dos des mains et parfois sur le visage. Petites taches de couleur brune, lisses, sans relief, de quelques millimètres à quelques centimètres de diamètre, leur pronostic est toujours bénin. Ces tâches peuvent également se développer chez le sujet âgé, lors d'inflammations ou de dérèglements hormonaux.

Elles sont considérées comme une disgrâce esthétique plus ou moins importante selon l'intensité de l'hyperpigmentation. La cause originelle est mal connue. Les conséquences physiologiques sont mieux étudiées: (1) augmentation de la synthèse de mélanine, (2) accélération du transfert de la mélanine des mélanosomes vers les kératinocytes et enfin (3) migration plus rapide des mélanocytes vers la surface cutanée.

### ETAT DE LA TECHNIQUE ANTERIEURE

Deux axes de traitement sont possibles: l'atténuation de l'hyperpigmentation, ou la destruction de la mélanine ou des tissus hyperpigmentés.

L'atténuation de l'hyperpigmentation peut être réalisée par des agents dépigmentants ayant une action pharmacologique, qui agissent sur les conséquences physiologiques. On peut citer à titre d'exemples le monométhyléther d'hydroquinone, le méquinol, la trétinoïne, ou encore l'acide kojique. La durée du traitement est longue et ses modalités d'application sont contraignantes (plusieurs applications par jour). La compliance est donc faible. De plus, le risque de récidive est très important puisque la cause originelle n'est pas traitée. Le patient réalise cette opération «à la maison», l'assistance du dermatologue n'étant pas nécessaire. Les effets secondaires et indésirables sont principalement des réactions inflammatoires localisées, des réactions allergiques aux principes actifs et une sensation de brûlure pour certains actifs.

Les dermocorticoïdes ont un potentiel dépigmentant certain, surtout lorsqu'ils sont appliqués sous pansement occlusif. Ils sont utilisés dans des crèmes à base d'hydroquinone, mais c'est davantage pour diminuer l'irritation induite par la préparation que pour en accroître l'efficacité. Le monobenzyléther d'hydroquinone est à proscrire. En effet, très puissant et peu maniable, il donne de fréquentes dépigmentations à distance de la zone traitée. Il a été par le passé responsable d'accidents cosmétiques sévères, avec des leuco-mélanodermies définitives. Certains l'utilisent encore dans les vitiligos étendus, pour compléter la dépigmentation des zones de peau saine ; cela nécessite deux applications par jour pendant plusieurs mois, à une concentration de 5 à 20%. Il peut être irritant ou allergisant.

La destruction de la mélanine ou plus généralement des tissus hyperpigmentés est réalisée par les techniques de cryothérapie c'est-à-dire l'application d'un froid intense pendant une courte période de temps, par le peeling, la micro-dermabrasion et par les lasers. Pour ces techniques, la durée de traitement est courte, les résultats sont satisfaisants et le risque de récidive est quasi nul. Mais le traitement est réalisé chez le dermatologue. Il existe de grandes disparités entre les différentes options de traitement au niveau des effets secondaires et indésirables. Ceux-ci se présentent principalement sous la forme d'une brûlure après l'application et d'un risque de cicatrice.

La cryothérapie est utilisée sur certaines lésions épidermiques, comme les lentigos actiniques. La technique est très variable suivant le matériel utilisé pour appliquer le froid. Les dermatologues se servent essentiellement de sprays pulvérisant de l'azote liquide sur la surface à détruire. Les sprays peuvent être ouverts ou fermés, l'azote étant alors projeté dans des cônes de néoprène dont la taille est adaptée à la surface à traiter. Cette technique est souvent précédée d'un curetage de la zone à traiter qui permet de réaliser un prélèvement pour examen anatomopathologique, de déterminer de façon plus précise les limites de l'extension et d'optimiser les résultats. Un ou plusieurs cycles de congélation - décongélation sont ainsi réalisés, en règle 2 cycles.

Une autre méthode consiste à appliquer l'azote liquide par le moyen de cryodes fermées dont la taille est adaptée à la surface cible. Un contrôle de la température intratissulaire à l'aide d'aiguilles, par thermocouple ou par impédancemétrie, est possible mais n'est pas réalisé systématiquement. L'existence d'un halo de congélation en périphérie de la zone cible et le temps de congélation et de décongélation sont utilisés pour apprécier la destruction tissulaire. Il s'agit d'une technique relativement simple, ambulatoire, qui permet de traiter des lésions multiples et qui n'est pas contre-indiquée chez les malades sous anticoagulants. L'évolution de la cryonécrose se fait sur plusieurs semaines avec la nécessité de pansements à renouveler. Elle peut donner lieu à des séquelles dyschromiques hypo- ou hyperpigmentées.

Lorsque les tâches cutanées sont très nombreuses ou très larges, un traitement à l'azote liquide s'avère trop violent et on peut préférer utiliser par exemple de la neige carbonique ou N20. Le brevet US 7,963959 décrit un dispositif automatisé piloté à l'aide d'un système d'acquisition d'images pour le traitement de nombreuses zones cutanées au moyen de divers fluides cryogéniques, dont des CFC. Ce dispositif est destiné à être mis en oeuvre dans un cadre médicalisé.

Les demandes de brevet FR 2 885 059 et FR 2 885 539 décrivent des dispositifs manuels pour appliquer un fluide cryogénique contenu dans une réserve d'aérosol sur une zone cutanée à traiter, via une buse d'éjection, la durée d'application étant contrôlée par une minuterie mécanique. Ces dispositifs sont conçus pour permettre un traitement en dehors d'un milieu hospitalier ou médicalisé. Toutefois les gestions du débit de fluide et de la durée d'application du fluide se sont avérées en pratique non maitrisées, très peu fiables et non reproductibles, de sorte que les effets locaux des applications de fluide cryogénique sont extrêmement disparates. En effet, ces applications donnaient lieu à des variations importantes de températures locales instantanées des zones traitées d'un essai à l'autre du fait de cette absence de reproductibilité, et ne présentaient pas la sécurité attendue avec ce type de dispositif à cause des risques de brulures importantes et de nécrose étendue.

Par ailleurs la mise en oeuvre des dispositifs de l'art antérieur produit un effet de lyse cellulaire non sélective, c'est à dire qu'ils produisent une nécrose de l'ensemble des populations cellulaires des tissus des zones traitées. En effet, leur nature même ou leurs mécanismes de fonctionnement ne permettent pas un contrôle précis de la dose délivrée et de la durée d'application du fluide cryogénique sur une zone déterminée, et par conséquent de la température obtenue sur la zone traitée. La plage des températures obtenues dans ledit tissu étant large ne permet pas d'obtenir une action sélective vis-à-vis des populations cellulaires présentes. En fait, l'homme du métier cherchait jusqu'à présent une action de nécrose cellulaire locale indifférenciée, et n'avait pas perçu l'intérêt à disposer d'un dispositif permettant un réglage très précis de la température à atteindre sur une zone cible.

### EXPOSE DE L'INVENTION

Au regard des techniques et produits utilisés actuellement, l'invention vise donc à proposer des moyens pour:
- Eliminer totalement les taches brunes et non pas atténuer leur couleur;
- Réduire drastiquement le temps de traitement avant résultat, par un effet immédiat;
- Simplifier la méthode de traitement de façon à obtenir l'élimination d'une tâche en une seule application;
- Améliorer la cicatrisation (absence de marque sur la peau après traitement);
- Augmenter la vitesse de re-pigmentation de la peau;
- Diminuer la douleur lors du traitement, ainsi que les rougeurs et oedèmes après le traitement;
- Activer la reconstruction et le renouvellement cellulaire.

Pour ce faire est proposé un procédé de traitement cosmétique selon la revendication 1.

Le traitement selon l'invention se caractérise par une sélectivité cellulaire ou cyto-sélectivité. Dans le cadre de la présente invention, on entend par action cyto-sélective ou cyto-sélectivité le fait d'agir sélectivement sur une population de cellules d'un tissu considéré, sans agir sur les autres populations de cellules. Le procédé de traitement selon l'invention atteint les mélanocytes situés au niveau de la couche basale ou jonction dermo-épidermique mais ne détruit pas les kératinocytes.

Pour la mise en oeuvre de ce procédé l'invention propose un dispositif selon la revendication 8.
Des modes d'exécution de l'invention sont définis par les revendications dépendantes.

### BREVE DESCRIPTION DES FIGURES

D'autres caractéristiques et avantages de l'invention ressortiront à la lecture de la description qui suit, en référence aux figures annexées, qui illustrent :
- la figure 1 est une vue schématique en coupe longitudinale d'un dispositif selon l'invention ;
- la figure 2 est une courbe expérimentale montrant l'effet thermique obtenu à l'aide d'un dispositif selon la figure 1.

### DESCRIPTION DÉTAILLÉE DE MODES DE REALISATION

La cryothérapie est une technique douloureuse en règle générale. Il a été rapporté que dans le traitement des verrues vulgaires, la cryothérapie est perçue comme une technique douloureuse chez 64% des patients traités par cryothérapie agressive, c'est à dire une application de froid d'une durée supérieure à 10 secondes. Dans le cas ou l'application du froid se fait pendant une période inférieure à 10 secondes, la douleur n'est plus perçue que par 44% des patients. Plus le temps d'application est faible, moins forte est la douleur. Cependant, une faible exposition au froid diminue considérablement l'efficacité du traitement puisque dans ce cas, seulement environ 30% des patients sont guéris. Il apparaît clairement une relation entre le temps d'application, l'efficacité du traitement par cryothérapie et la douleur.

La réfrigération d'un tissu conduit à des changements d'état physique et, selon les conditions d'application du froid, à sa préservation ou au contraire à sa destruction. Le choc thermique appliqué dans le cadre de la présente invention est un très grand abaissement de la température en un minimum de temps. La destruction tissulaire s'obtient par une congélation très rapide, suivie d'un réchauffement lent durant lequel l'action destructrice va se prolonger. L'abaissement très brutal de la température engendre, avant même que le tissu soit solidifié, une micro-cristallisation de l'eau intracellulaire; ces microcristaux induisent altérations membranaires, dénaturation de protéines de structure et enzymatiques, surconcentrations ioniques, toutes conditions conduisant à un effet délétère sur la cellule. Une recristallisation de l'eau en excès pendant la phase de réchauffement majore encore la lyse cellulaire. Dans les conditions normales la température cutanée est aux alentours de 34°C. C'est cette température qui doit être abaissée un maximum en un minimum de temps.

Les mélanocytes sont situés au niveau de la jonction dermo-épidermique, migrent au cours des quatre stades de leur maturité vers la surface de la peau, c'est à dire la couche superficielle de l'épiderme. Ces mélanocytes contiennent des mélanosomes, qui sont des vacuoles contenant la mélanine. La maturation des mélanosomes et la concentration en mélanine se fait à l'intérieur des mélanocytes. Puis les mélanosomes sont transférés au niveau des dendrites des mélanocytes et vers les kératinocytes, qui les intègrent dans leurs structures cellulaires. Ils se placent au dessus du noyau pour le protéger des rayonnements UV. Puis les mélanosomes sont dégradés par des enzymes. La mélanine libérée est éliminée au niveau de la surface épidermique par la desquamation de la couche cornée et dans le derme par voie lymphatique.

Les hyperpigmentations résultent d'un trouble de la mélanogenèse, avec activité accrue au niveau des mélanosomes et parfois d'un transfert plus important du pigment dans les kératinocytes de la couche de Malpighi, ou d'une accumulation de mélanine dans le derme. Il s'agit donc d'une hypermélanocytose, située au niveau de la couche basale : l'hypermélanocytose se caractérise par l'augmentation du nombre de mélanocytes, ou une augmentation de la synthèse de mélanine par les mélanocytes. La cellule clé et par voie de conséquence la cellule cible, est donc le mélanocyte. Les kératinocytes ne doivent pas être touchés par un traitement car ils constituent la protection du tissu épidermique contre le rayonnement UV.

L'épaisseur de l'épiderme varie en fonction de la zone concernée, de 0,02 mm au niveau de la peau du visage à 5 mm au niveau de la plante des pieds. Son épaisseur moyenne est de 0,1 mm. Au niveau des mains (dessus des mains), les mélanocytes sont situés à 0,1 mm de la surface de la peau. Dans un épiderme soumis au froid les mélanocytes sont viables s'ils sont soumis à une température comprise entre 0 et -4°C. Entre -4°C et -7°C, on observe une lyse des granules contenant les pigments, c'est-à-dire les mélanosomes contenant la mélanine, et par la suite un début de digestion enzymatique de la mélanine dans les mélanocytes et dans les kératinocytes dans la couche profonde de l'épiderme, près de la couche basale. Entre -7°C et -30°C, on observe une disparition des mélanocytes et au delà pas de réapparition de mélanocytes (troubles de dépigmentation). La destruction des kératinocytes intervient pour des températures inférieures à -20°C, avec une destruction très importante des populations de mélanocytes fortement différenciés.

Les inventeurs en concluent que l'efficacité du traitement sur l'hyperpigmentation due aux mélanocytes, sans dommages importants pour les kératinocytes, se situe dans une plage comprise entre -4°C et -15°C et de préférence entre -4°C et -10°C. Le froid délivré à la surface de la peau doit y générer une température comprise de préférence entre -4°C et -10°C pour agir sur les mélanocytes et la mélanine de manière cyto-sélective. Ainsi, l'action d'une température comprise entre -4°C et -10°C permet d'agir sur la mélanine et les mélanocytes, sans endommager les kératinocytes, selon un principe de cytosélectivité, c'est à dire une sélectivité cellulaire. La barrière bénéfice (action sur la mélanine et le mélanocyte entre -4° et -10° C.) versus risque (destruction du kératinocyte en dessous de -20° C) est donc importante et va dans le sens d'une grande sécurité d'utilisation du traitement cosmétique et d'une absence d'effets secondaires (douleur et cicatrice) selon l'invention.

Certains fluides réfrigérants ont des propriétés de condensation d'eau plus marquées que d'autres. C'est le cas notamment du diméthyl-éther, fluide cryogénique utilisé dans certains produits de cryothérapie pour traiter des verrues. Lorsque ce mélange arrive dans un embout en mousse, il y a condensation de vapeur d'eau contenue dans l'air dûe au contact de la mousse froide avec l'air ambiant. On peut observer systématiquement la formation de gouttelettes qui gèlent aussitôt sur la surface de l'embout lorsque celui-ci est maintenu à l'air libre. Ce fluide demeure longtemps à l'état liquide sur la peau, il s'évapore lentement, emprisonné dans l'embout. Lors de l'application de cet embout sur la peau, il y a ainsi une certaine quantité d'eau qui est déposée à l'endroit de l'application, et cette humidité renforce très nettement la sensation de douleur. Par contre, le difluoroéthane, code 152A, s'évapore très vite, ce qui permet au froid de pénétrer plus rapidement dans la peau. Cette vitesse d'évaporation et une pulvérisation directe sans contact sur la peau évitent la condensation d'eau et son "emprisonnement". Le difluoroéthane (152A) a été choisi pour sa température d'ébullition de -24°C, et sa faible chaleur latente d'évaporation (160 KJ/kg), afin de produire un fluide très volatile pour éviter la formation des gouttes sur la peau, et, par voie de conséquence la sensation de douleur, et apporter suffisamment de froid pour être efficace.

Lorsque la peau est soumise à une source de froid appliquée à sa surface, ceci engendre un abaissement rapide de température: en l'espèce l'application d'un jet de difluoroéthane sans contact avec la peau produit une variation de température superficielle de l'ordre de 10 à 20°C par seconde. Il permet donc de diminuer la température du tissu d'environ 34°C vers -5°C à-10°C, soit environ 40°C de variation, en un peu plus de 2 secondes, en fait 2,5 secondes à 3 secondes, car il y a des pertes thermiques.

Une autre mode de réalisation de l'invention utilise du gaz 134A, c'est-à-dire le tétrafluoroéthane.

Le changement de température, progresse à raison de 0,5 à 1 mm par seconde à travers les couches de la peau. En conséquence, la température de la couche basale engendrée par cette source de froid est identique à la température à la surface de la peau en environ 0,2 secondes. Donc, en moins de 1 seconde, la température des mélanocytes au niveau de la couche basale sera identique à celle à la surface cutanée. Un temps de pulvérisation du fluide cryogénique de l'ordre de 3 secondes est donc cohérent avec l'effet recherché.

Selon un mode d'exécution préféré, le procédé est mis en oeuvre à l'aide d'un diffuseur directif de difluoroéthane (152A) avec système de temporisation préréglée à trois secondes, dont les principaux éléments sont décrits ci-dessous.

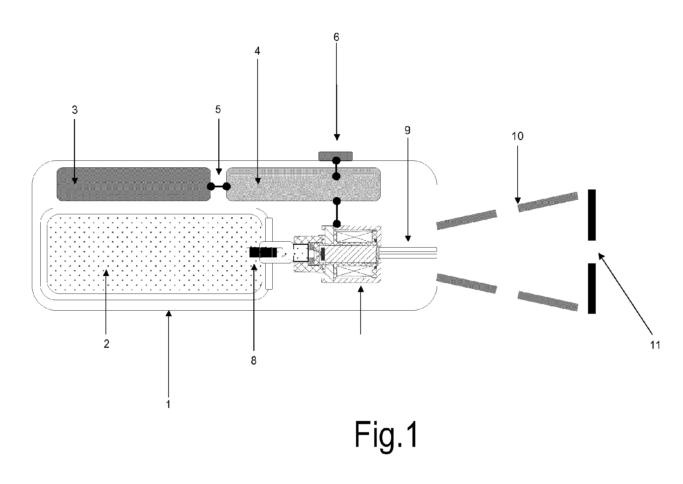

Comme le montre la figure 1, les éléments constituant le dispositif sont entourés d'un carter 1. Ce carter contient une cartouche 2 de gaz cryogénique, de préférence du 152A, éventuellement du 134A (tétrafluoroéthane). La cartouche 2 est reliée à une électrovanne 7 par l'intermédiaire d'un stem 8 permettant le passage du fluide de l'intérieur de la cartouche vers l'extérieur. En position de repos du dispositif, le stem 8 laisse pénétrer le fluide dans la chambre amont de l'électrovanne 7. Le carter 1 contient une source d'énergie, par exemple sous forme de pile(s) électrique(s) 3. La source d'énergie est reliée via un contacteur 5 à un système électronique de temporisation 4 permettant le passage de fluide dans l'électrovanne 7 pendant un temps prédéterminé. Le système électronique de temporisation 4, et par voie de conséquence l'électrovanne 7, est actionné par un bouton de déclenchement 6. En aval de l'électrovanne 7 est agencé un gicleur 9. Cet ensemble permet un prélèvement de fluide cryogénique et son éjection via le gicleur pendant une durée prédéterminée, par exemple trois secondes, précise à 0,1 seconde près. La précision de la temporisation de l'électrovanne est nécessaire pour garantir une action cyto-sélective sans atteindre des températures délétères pour les kératinocytes.

Le gicleur 9 débouche dans une buse 10 agencée à l'extérieur du carter 1. La buse 10, qui est conique dans le mode d'exécution représenté sur la figure 1, se termine par un embout 11 avec un orifice permettant le contact entre le jet de gaz froid et une zone cible de la peau. Cet orifice peut présenter une aire qui correspond au diamètre des lentigos les plus étendus que l'on puisse traiter dans le cadre d'un procédé cosmétique sans risque de confusion avec un éventuel mélanome. L'orifice peut donc présenter par exemple une forme circulaire avec un diamètre de 6 mm. De la sorte, une tache cutanée peut être traitée en une seule application. La forme conique de la buse, sa longueur de l'ordre de 35 mm, ses ouvertures latérales, et son embout ont été étudiés pour que le gaz se diffuse à une distance appropriée sur une zone précise des tissus dans des conditions de température optimum pour garantir l'action cyto-sélective. En effet les expérimentations montrent qu'en diminuant cette distance entre la sortie du gicleur et les tissus, l'impact exercé sur les tissus par le jet de gaz reste trop important, le fluide ne s'étant pas encore totalement transformé à l'état gazeux, et le niveau de température cutanée s'abaisse plus fortement que celui exigé pour une action cyto-sélective. De même si on augmente la distance de jet du gaz entre le gicleur et le tissu, la température n'arrive pas à descendre à des niveaux suffisamment froids, aucune action efficace ne peut être obtenue. Les ouvertures latérales de la buse permettent à un surplus du gaz de s'échapper, ce qui améliore la juste délivrance d'une dose spécifique de gaz pour une action cyto-sélective.

Dans le mode de réalisation de la figure 1 le fluide cryogénique passe successivement de la cartouche 2 et du stem 8, dont le diamètre de sortie peut varier de 3 à 4 mm, dans l'électrovanne, dont l'entrée et la sortie peuvent varier de 0,15 mm à 0,25 mm. Le fluide cryogénique passe dans une chambre de l'électrovanne 7, qu'il suit sur une longueur pouvant être comprise entre 10 à 20 mm. A la sortie de cette électrovanne, il entre dans un tube en métal formant le gicleur 9, dont la longueur peut être comprise entre 5 et 15 mm avec un diamètre intérieur de 0,15 mm à 0,25 mm. Ce dernier élément long et particulièrement étroit, dans lequel circule le fluide cryogénique avant sa détente à l'air ambiant produisant l'effet cryogénique, contribue à réduire sa vitesse initiale et favorise la projection de ce gaz sur le tissu à traiter.

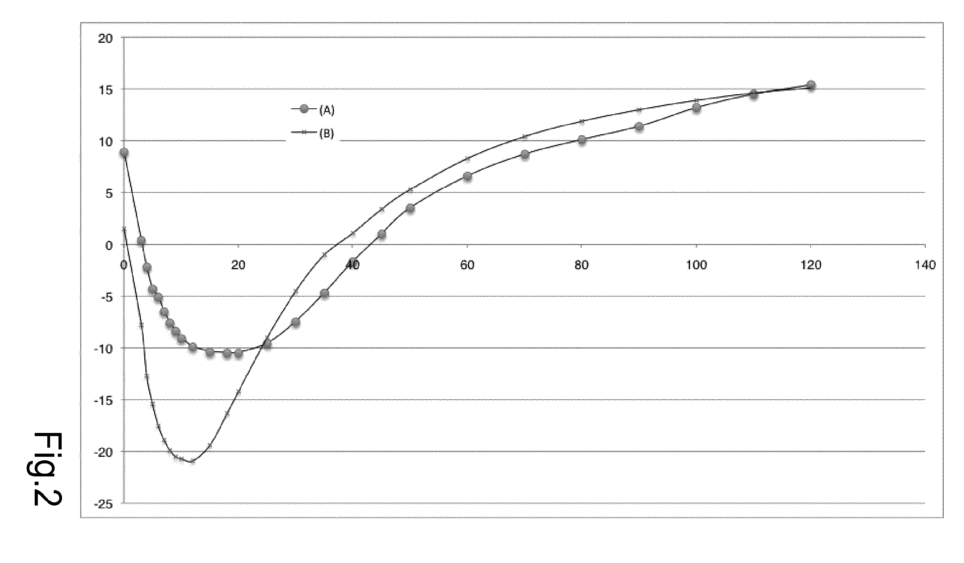

Dans le cadre de l'invention, il a été constaté que des modifications du diamètre et de la longueur du gicleur 9 modifient considérablement le débit du gaz, et ces modifications de débit jouent un rôle important en combinaison avec des modifications de temps d'ouverture de l'électrovanne sur la température de la surface de la peau atteinte. En particulier, et selon un mode d'exécution particulièrement préféré, une longueur de 7 mm pour un diamètre de 0,17 mm du gicleur, en combinaison avec une ouverture de l'électrovanne de trois secondes permet d'obtenir une plage de température sur un tissu conduisant à une action cyto-sélective. La figure 2 illustre ce qui précède en montrant la température de surface de la peau obtenue après trois secondes d'ouverture de l'électrovanne avec (A) et sans (B) le gicleur 9. L'absence du gicleur ne permettrait pas d'atteindre la plage de température permettant la cyto-sélectivité puisqu'on atteindrait une température délétère pour les kératinocytes.

### EXEMPLE

Une étude a été réalisée de manière à valider le dispositif décrit ci-dessus. L'étude a consisté à appliquer un gaz cryogénique 152A sur des sujets présentant des taches brunes sur le dos de la main. L'étude a été réalisée sur 4 sujets, deux hommes et deux femmes, JMPAT, CDEN, AMAH et YPHIL, âgés de 50, 57, 59 et 55 ans. La durée d'application du gaz cryogénique a été réglée à 3 secondes.

Les sujets présentaient les caractéristiques suivantes:
- JMPAT: présence d'une tache brune fortement marquée au niveau de la main droite, au niveau de l'index.
- CDEN: présence d'une tache brune marquée au niveau de la main droite, situé entre l'index et l'auriculaire.
- AMAH: présence de deux taches brunes de taille importante, situées au niveau de la main droite.
- YPHIL: présence de deux taches brunes, presque accolées l'une à l'autre au niveau de la main droite.
Les résultats montrent que le dispositif permet d'éliminer les taches brunes après une seule pulvérisation de 3 secondes par tache. En effet, les taches brunes traitées ont totalement disparu chez tous les sujets. Certains des sujets ayant participé à l'étude avaient déjà été traités par un dermatologue sur d'autres taches brunes similaires du dos de leur main. Le traitement avait consisté en l'application d'une cryothérapie traditionnelle à base d'azote ou de diméthyléther.
Contrairement à ces traitements, l'application du froid cryogénique par le dispositif objet du brevet n'a pas induit chez ces sujets de sensation de douleur, alors qu'une forte douleur avait été ressentie lors du traitement effectué par le dermatologue. De plus, on note l'absence d'inflammation importante, et de destruction tissulaire conduisant à des marques sous la forme de cicatrices ou d'hypo-pigmentation, ce qui n'avait pas été non plus le cas lors du traitement chez le dermatologue.
Ces observations confirment que le dispositif présente bien un effet cyto-sélectif, alors que la cryothérapie traditionnelle à base d'azote ou de diméthylether est sans effet cyto-sélectif. En effet, le dispositif grâce à son action cyto-sélective ne produit ni la destruction par nécrose de l'ensemble du tissu, ni la destrcution des kératinocytes et donc n'induit pas les phénomènes observés avec la cryothérapie traditionnelle à base d'azote telle que pratiquée au cabinet de dermatologie (inflammation, douleur importante, cicatrice, hypo-pigmentation).

Bien que présentée sur la figure 1 en forme de diffuseur oblong agencé pour contenir une cartouche de difluoroéthane, l'invention peut s'appliquer à toute forme de diffuseur de fluide cryogénique ayant une température d'ébullition entre-20°C et -65°C et une chaleur latente d'évaporation comprise entre 1 et 500 KJ/Kg, muni d'un dispositif de focalisation approprié sur une zone cutanée et d'un dispositif de temporisation préréglée ou réglable. De plus, bien que la description qui précède s'est, pour des raisons de concision, bornée à décrire un type de dispositif additionnel sous forme de buse conique, l'invention est applicable à tout type de dispositif additionnel qui permet de manière fiable l'établissement sur une aire prédéterminée de peau d'une température comprise entre -4°C et -10°C.

Comme cela a été décrit ci-dessus, la présente invention propose un procédé de traitement cosmétique de tissus cutanés, destiné à obtenir une action cyto-sélective mélanocytes versus kératinocytes. Comme mentionné déjà plus haut, on entend par action cyto-sélective ou cyto-sélectivité le fait d'agir sélectivement sur une population de cellules d'un tissu considéré, sans agir sur au moins une autre ou les autres populations de cellules. La présente invention pourrait s'appliquer à d'autres types de populations de cellules dans d'autres tissus biologiques, le terme population de cellules étant compris dans son sens le plus large, c'est à dire un ensemble de cellules qui présentent les mêmes caractéristiques par exemple un type de cellules, une lignée cellulaire, une souche de cellules, procaryotes ou eucaryotes, de toutes origines, humaines, animales ou végétales. Parmi les types de cellules susceptibles d'être concernées on peut citer de manière non limitative les cellules souches et les cellules des tissus épithéliums, glandulaires, musculaires, conjonctifs, osseux, adipeux ou nerveux ainsi que les cellules du sang. De plus, le procédé peut s'appliquer également à des éléments constitutifs des cellules c'est-à-dire des organites cellulaires, notamment et de manière non limitative aux nucléole, noyau, ribosome, vésicule, réticulum endoplasmique, appareil de Golgi, cytosquelette, mitochondries, vacuoles, cytosol, lysosomes, centrosome et membrane plasmique. Enfin, le procédé peut s'appliquer également à des virus, des molécules, à des organismes vivants de petite taille tels que les parasites ou autres corps exogènes pour un organisme donné. Un procédé du type décrit plus haut est susceptible de s'appliquer à condition que l'on puisse déterminer une plage de température délétère pour la population de cellules, de structures ou d'organismes cible, et dans laquelle plage une autre population ou le tissu environnant n'est pas impacté de manière notable ou rédhibitoire.

Les domaines d'application de l'invention peuvent être variés, et ne sont pas limités aux domaines de la cosmétique et de la pharmacie. Dans le domaine thérapeutique, une d'application potentielle de l'invention concerne les mélanomes. Le mélanome est un cancer de la peau ou des muqueuses, développé aux dépens des mélanocytes (tumeur mélanocytaire). Une action cyto-sélective telle que proposée par l'invention permettrait d'éliminer la tumeur sans affecter les tissus, populations de cellules, ou éléments constitutifs environnants.

## Revendications

1. Procédé de traitement cosmétique de taches brunes cutanées, destiné à faire disparaître au moins une desdites taches brunes située dans une zone des mains, du visage, des membres ou du décolleté d'un sujet atteint de telles hyperpigmentations cutanées, **caractérisé en ce qu'**il comprend une étape d'application d'un jet de fluide cryogénique sur ladite zone amenant ladite tache à une température comprise entre -4°C et -15°C.

2. Procédé de traitement selon la revendication 1, **caractérisé en ce que** ladite température est comprise entre -4° et -10°C.

3. Procédé de traitement selon la revendication 1 ou 2, **caractérisée en ce que** le dit fluide cryogénique est choisi parmi les fluides ayant une température d'ébullition sous pression atmosphérique entre -20°C et-65°C.

4. Procédé de traitement selon l'une des revendications 1-3, **caractérisé en ce que** le dit fluide cryogénique est le difluoroéthane 152A.

5. Procédé de traitement selon l'une des revendications 1-3, **caractérisé en ce que** le dit fluide cryogénique est le tétrafluoroéthane 134A.

6. Procédé de traitement selon l'une des revendications précédentes, **caractérisé en ce que** la durée d'application dudit jet de fluide cryogénique est comprise entre deux et quatre secondes.

7. Procédé de traitement selon l'une des revendications précédentes, **caractérisée en ce que** le dit traitement comprend une seule application de fluide cryogénique par tache.

8. Procédé de traitement selon l'une des revendications précédentes, **caractérisé en ce que** le jet de fluide cryogénique passe par un dispositif collimateur limitant l'impact dudit jet sur la peau du sujet à une zone de moins de 30 mm².

9. Dispositif pour mettre en oeuvre un procédé selon l'une quelconque des revendications précédentes, comprenant un réservoir (2) de fluide cryogénique, une vanne permettant le passage du fluide cryogénique de l'intérieur dudit réservoir vers une buse (9), **caractérisée en ce que** ladite vanne est une électrovanne (7) et que le dit dispositif comprend un système électronique de temporisation (4) permettant l'ouverture de l'électrovanne pendant une durée prédéterminée précise à 0,1 seconde près.

10. Dispositif selon la revendication précédente, **caractérisé en ce que** à la sortie de l'électrovanne sont agencés des moyens gicleurs (9) de limitation du jet de fluide cryogénique.

11. Dispositif selon la revendication 10, **caractérisé en ce que** les moyens gicleurs comprennent un tube gicleur dont la longueur est comprise entre 5 et 15 mm et de diamètre intérieur entre 0,15 et 0,25 mm.

12. Dispositif selon l'une des revendications 9-11 **caractérisé en ce que** ses composantes sont ajustées de façon à exercer une action cytoselective au sein du tissu traité.
